# EUROPÄISCHE PATENTSCHRIFT

(11) **EP 2 658 610 B1**
(45) Veröffentlichungstag und Bekanntmachung des Hinweises auf die Patenterteilung: **04.05.2016**
(21) Anmeldenummer: 11831813.8
(22) Anmeldetag: 24.11.2011
(51) Int. Cl.: A61Q 19/00, A61Q 19/08, A61K 8/06, A61K 8/37, A61K 8/67, A61K 8/92

(54) **KOLLOIDALES TRÄGERSYSTEM MIT PENETRIERENDEN EIGENSCHAFTEN ZUM EINSCHLIESSEN LIPOPHILER WIRKSTOFFE UND ÖLE FÜR DIE TOPISCHE ANWENDUNG**
COLLOIDAL CARRIER SYSTEM WITH PENETRATION PROPERTIES FOR ENCAPSULATING LIPOPHILIC ACTIVE AGENTS AND OILS FOR TOPICAL USE
SYSTÈME SUPPORT COLLOÏDAL PRÉSENTANT DES PROPRIÉTÉS DE PÉNÉTRATION POUR L'ENCAPSULAGE D'AGENTS ACTIFS LIPOPHILES ET D'HUILES POUR L'APPLICATION TOPIQUE

(30) Priorität: 28.12.2010 DE 102010056192
(43) Veröffentlichungstag der Anmeldung: 06.11.2013
(73) Patentinhaber: Blume, Gabriele, 36396 Steinau an der Strasse (DE)
(72) Erfinder: Blume, Gabriele, 36396 Steinau an der Strasse (DE)
(74) Vertreter: advotec.
(86) Internationale Anmeldenummer: PCT/DE2011/002026
(87) Internationale Veröffentlichungsnummer: WO 2012/089184

(56) Entgegenhaltungen:
- WO-A1-2006/087156
- US-B1- 6 419 946
- DATABASE WPI Thomson Scientific, London, GB; AN 2010-P72148 XP002695563, "Composite nanoemulsion for recovering and desensitizing sensitive skin, comprises isopropyl myristate, polyglyceryl-3-oleate, distilled water, citric acid, sodium citrate, metallothionein, Chrysanthemum extract, oxymatrine and vitamin E", & CN 101 869 539 A 27. Oktober 2010 (2010-10-27)
- JUSTAS BARAUSKAS ET AL: ""Sponge" Nanoparticle Dispersions in Aqueous Mixtures of Diglycerol Monooleate, Glycerol Dioleate, and Polysorbate 80", LANGMUIR, Bd. 22, Nr. 14, 1. Juli 2006 (2006-07-01), Seiten 6328-6334, XP055059935, ISSN: 0743-7463, DOI: 10.1021/la060295f

## Beschreibung

Die Erfindung betrifft die Verkapselung von schwer löslichen lipophilen Wirkstoffen und Ölen in Form eines kolloidalen Systems (Tröpfchen).

Die Verkapselung von lipophilen Wirkstoffen in Teilchen mit einer Größe im Submikrometerbereich - Öl in Wasser Nano- bzw. Mikroemulsionen - ist seit vielen Jahren bekannt. Solche Emulsionen werden insbesondere in der Kosmetik und Dermatologie häufig eingesetzt.

Mikroemulsionen unterscheiden sich von den klassischen Emulsionen durch ihre sehr geringe Tröpfchengröße zwischen 10 und < 100 nm. Das sichtbare Licht kann an ihnen nicht gestreut werden, sodass die Mikroemulsionen transparent wie Wasser erscheinen.

So wird im Patent DE 100 54 919 (A1) eine Mikroemulsion mit einer Tröpfchengröße von ca. 20 nm beschrieben, die zu Verkapselung der lipophilen Phase (40% Gew.%) eine Mischung aus Tensid (Tween 80 = Poyoxyethylen-20-sorbitan-monooleate) und Cotensid (Poloxamer 331) in einer Menge von bis zu 30 Gew.% benötigt. Die Mikroemulsion ermöglicht den Transport des eingeschlossenen Wirkstoffes (hier Lidocain) in die Haut.

Eine weitere Beschreibung einer Mikroemulsion (US 2009/0324727 A1) beruht ebenfalls auf dem Tensid Tween 80, und als Coemulgator wird diesmal Lecithin verwendet. Auch hier wird eine hohe Emulgatormenge (bis 30%) zum Verkapseln des Öles (15%) unter Zusatz eines mehrwertigen Alkohols gebraucht.

Aus dermatologischer Sicht ist der oft recht hohe Tensidgehalt der Mikroemulsionen als nachteilig zu bewerten, da er die Gefahr des Auftretens von Hautirritationen in sich birgt und beim Aufbringen auf die Haut ein klebriges Gefühl hervorruft. Ihr Formulierungsbereich ist außerdem im Allgemeinen sehr begrenzt (Anwendung vorwiegend als Sprühemulsion oder wässeriges Gel).

Nanoemulsionen (Teilchengröße 50 - 250 nm) sind metastabile Öl in Wasser-Emulsionen bei denen ein lipophiler - meist flüssiger - Kern von einer Schale aus amphiphilen grenzflächenaktiven Molekülen umgeben ist. Diese Emulsionen werden in Kosmetik und Pharmazie vorwiegend zur Solubilisierung der schwer löslichen lipophilen Komponenten in Wasser und zu deren Stabilisierung eingesetzt. Nanoemulsionen - zum Teil auch transparente - mit kleiner Tröpfchengröße lassen sich nur mittels eines Hochdruck-Homogenisators herstellen.

L'Oreal beschreibt in ihren Patenten EP 728 460 (B1) und EP 1 353 629 (B1) transparente Nanoemulsionen mit einer Tröpfchengröße < 100 nm auf Basis von flüssigen, nichtionischen amphiphilen, grenzflächenaktiven Substanzen. Diese nicht ionischen Tenside sind der Gruppe der Polyoxyethylenglykol-Ester zuzuordnen (PEGₙ-Ester). Auch in den folgenden beiden Druckschriften wird dieser Typ von Emulgator als ein Bestandteil für die Bildung der Schale von Nanoemulsionen eingesetzt (EP 1 839 644 A3 und US 2009/0208541 A1). Keine der eben zitierten Schriften beschreibt aber den Transport von lipophilen Wirkstoffen mittels der kolloidalen Teilchen in die tieferen Hautschichten.

Die Firma Ciba hat mit ihrem Produkt "Tinoderm E" eine transparente Nanoemulsion auf den Markt gebracht, die mit ihrer Teilchengröße von 20-40 nm den Transport vom im Ölkern befindlichen Vitamin E in die Haut ermöglicht. Die Penetrationseigenschaften werden der sehr geringen Teilchengröße zugeordnet, die dem Bereich der kleinsten Zwischenabstände in den oberen Hautschichten entspricht. Auch hier enthält das Emulgatorengemisch für die Herstellung der Nanoemulsion einen Polyoxyethylenglykol-Ester.

In der Kosmetik geht der Trend aber zu PEG-freien (Polyoxyethylenglycol-freien) Formulierungen, weshalb möglichst Nanoemulsionen mit natürlichen, pflanzlichen Emulgatoren hergestellt werden sollen.

Die Firma L'Oreal beschreibt in einer Vielzahl von Patenten "Nanoemulsionen auf Basis eines grenzflächenaktiven Stoffes und mindestens ein Öl mit einer Molmasse über 400, wobei das Gewichtsverhältnis Menge Öl zu Menge grenzflächenaktiven Stoffes bei 2 bis 10 liegt. Die Größe der Ölkügelchen ist sehr gering und liegt unter 100 nm, meist im Bereich von 40 bis 60 nm.

Bei EP 1 010 413 (B1) sind die grenzflächenaktiven Stoffe: Zuckerfettsäureester oder Zuckerfettalkoholether; bei EP 1 010 414 (B1) sind die die grenzflächenaktiven Stoffe: Mischester von Fettsäuren oder Alkohol, von Carbonsäuren und Glyceryl; bei EP 1 010 415 (B1) sind die grenzflächenaktiven Stoffe: Fettsäureester von ethoxylierten oder von nicht ethoxylierten Sorbitan; bei EP 1 010 416 (B1) sind die grenzflächenaktiven Stoffe: Fettsäureester vom Glycerin; bei EP 1 013 338 (B1) sind die grenzflächenaktiven Stoffe: Fettsäureester von Phosphorsäure; bei EP 1 016 453 sind die grenzflächenaktiven Stoffe: ethoxylierte Fettalkoholether oder ethoxylierte Fettsäureester; bei EP 1 020 219 sind die grenzflächenaktiven Stoffe: Alkylethercitrat und bei Patent EP 1 025 898 sind die grenzflächenaktiven Stoffe: alkoxylierte Alkenylsuccinate und alkoxylierte Alkenylsuccinate der Glukose.

Bei all diesen Patenten liegt das gleiche Anwendungsbeispiel zugrunde - ein Fluid zum Abschminken - mit immer gleicher Zusammensetzung und gleichen Gewichtsanteilen, nur der eine Emulgator (grenzflächenaktiver Stoff) variiert.

In all diesen oben genannten Patenten werden keine Angaben über den Transport der in den Tröpfchen evt. verkapselten Wirkstoffe in die Haut aufgeführt und deren Bioverfügbarkeit in der Epidermis.

Den Transport von Wirkstoffen in die Haut mit Hilfe eines kolloidalen Trägersystems (Größe zwischen 100 und 200 nm) wird nur im amerikanischen Patent US 2008/0193393 beschrieben. Das Trägersystem beruht auf einem Ölkern, der den gelösten lipophilem Wirkstoff enthält und von einer Membran aus Phospholipiden und Fettsäuren umgeben ist. Dennoch muss hier hervorgehoben werden, dass bei den Penetrationsversuchen die Formulierung okklusiv aufgetragen wurde (unter Verwendung von Parafilm), was nicht der Realität entspricht. Okklusion führt zu einem Feuchtigkeitsstau in der Hornschicht der Haut (Stratum corneum) und infolge dessen zu Quellung. Das erhöhte Wasserangebot erleichtert das Eindringen vieler pharmazeutischer Wirkstoffe = Wirkstoffpenetration (Wikipedia).

WO 2006/087156 A1 beschreibt Nanoemulsionen, welche Lipoaminosäuren sowie Ester von Fettsäuren mit Mono-, Di- und/oder Polyglyceriden enthalten.

Es ergab sich somit die Aufgabe, ein gut penetrierendes kolloidales Trägersystem mit erhöhter Verkapselung an lipophilen Wirkstoffen bereitzustellen, das einfach zu produzieren ist.

Gelöst wird die Aufgabe durch das erfindungsgemäße kolloidale Trägersystem, das die folgenden Bestandteile enthält:
- einen flüssigen Lipidkern
- eine den Kern umgebende kontinuierliche Schale, gebildet aus einem anionischen, membranbildenden Emulgator und mindestens einem nicht ionischen einkettigen Coemulgator, ausgewählt aus der Gruppe von Diglyceryl-Monooleate, Diglyceryl-Monolinoleate, Polyglyceryl-Monooleate, Polyglyceryl-Monolinoleate, wobei es sich bei dem membranbildenden, natürlichen anionischen Emulgator um Glyceryl Citrate / Lactate / Linoleate / Oleate handelt.

Die Aufgabe wird ferner gelöst durch eine Zubereitung, enthaltend das kolloidale Trägersystem wie im Folgenden beschrieben.

Das erfindungsgemäße Trägersystem liegt in einer wässerigen Dispersion vor, sodass die eingeschlossenen lipophilen Wirkstoffe in höherer Konzentration einfach in wasserbasierte Formulierungen eingearbeitet werden können, z B. Sprühformulierungen, Gele oder Lotionen und O/W Emulsionen. Besonders hervorzuheben ist, dass diese Teilchen eine Penetration der eingeschlossenen Wirkstoffe in die Haut ermöglichen und somit als kolloidales Wirkstoffträgersystem fungieren.

Ferner stabilisiert dieses kolloidale System eingeschlossene sensitive Moleküle wie zum Beispiel Vitamine vor der Degradation. Die Kombination aus einem membranbildenden anionischen natürlichen Emulgator mit einem im Anspruch 1 genannten Coemulgator (Stabilisator) führt zu einer hohen Verkapselung an lipophilen Wirkstoffen im wässerigen Medium.

Das erfindungsgemäße kolloidale Trägersystem weist eine negative Ladung auf und hat vorzugsweise eine Größe von 100 bis 200 nm.

Ein solches kolloidales Trägersystem mit einer Tröpfengröße von 100 bis 200 nm, die einen Transport der verkapselten lipophilen Komponenten in die Epidermis ermöglicht, ist aus dem Stand der Technik nicht bekannt.

In einer bevorzugten Ausführungsform enthält die erfindungsgemäße Zubereitung noch einen Stabilisator (z.B. wasserlöslichen Alkohol oder Polyol) und ist damit als konservierungsmittelfrei anzusehen.

Die das kolloidale Trägersystem enthaltende wässerige Zubereitung kann selbst als Kosmetikum / Pharmazeutikum eingesetzt werden, je nach enthaltenem Wirkstoff. Darüber hinaus lässt die das kolloidale Trägersystem enthaltende wässerige Zubereitung sich in die Wasserphase einer weiteren Zubereitung, wie einer Emulsion, Lotion, Creme oder unterschiedliche Formen von Gelen, einarbeiten oder kann selbst verdünnt mit einem zugesetzten Verdicker als Sprühformulierung angewendet werden.

Die vorliegende Erfindung weist eine hohe Verkapselungseffizienz der lipophilen Stoffe auf. Die erfindungsgemäße Zubereitung enthält den flüssigen Lipidkern mit Vorteil in einer Menge von 15 - 40 Gew.-%, bevorzugt von 20 - 35 Gew.-% bezogen auf die Gesamtmenge der Zubereitung.

Weitere Vorteile der erfindungsgemäßen Zubereitung sind die hohe Stabilität der Komposition gegen Phasentrennung, ein gutes Hautgefühl und die leichte Verteilbarkeit auf der Haut.

Neben dem membranbildenden pflanzlichen anionischen (lamellare Strukturen bildenden) Emulgator wird ein weiterer pflanzlicher, nichtionischer einkettiger Coemulgator eingesetzt, die beide als hydrophoben Teil langkettige Fettsäuren (z.B: Palmitin-, Stearin-, Öl- und / oder Linolsäure) enthalten.

Diese Emulgatoren mit langer Alkylkette zeichnen sich durch eine hohe Hautverträglichkeit aus und können kosmetische und pharmazeutische Effekte erzielen (z. B: Erhöhung der Hautglätte, Reduzierung von Metalloproteasen, Reduzierung von Hautunreinheiten).

Als Coemulgatoren werden bevorzugt Diglyceryl-Monooleate eingesetzt.

Die Menge an membranbildendem Emulgator Glyceryl Citrate / Lactate / Linoleate / Oleate (Imwitor 375) in den erfindungsgemäßen Zusammensetzungen beträgt zwischen 3 und 6 Gew.-% und die des Coemulgators (z. B. Diglyceryl-Monooleate Nikkol DGMO) zwischen 1 und 3 Gew.-%. Beide Komponenten bilden die Schale, die den flüssigen Lipidkern umgibt.

Der Ölkern enthält vorzugsweise natürliche pflanzliche Öle und in ihm gelöste lipophile Wirkstoffe. Dabei beträgt der Ölkern bevorzugt zwischen 20 und 35% bezogen auf die Gesamtformulierung und die der membranbildenden Komponenten zwischen 4 und 8%.

Unter Fettstoffen sind vorzugsweise natürliche und synthetische kosmetische Ölkomponenten sowie natürliche und synthetische Wachse zu verstehen, die erfindungsgemäß bei Raumtemperatur flüssig sind. Diese Ölkomponenten sind physiologisch akzeptabel und hautfreundlich.

Bei dem Fettstoff kann es sich insbesondere um ein unpolares oder polares flüssiges Öl - das natürlich oder synthetisch sein kann - handeln. Die Ölkomponente kann insbesondere ausgewählt sein aus pflanzlichen Ölen, insbesondere Sonnenblumenöl, Olivenöl, Sojaöl, Rapsöl, Mandelöl, Orangenöl, Weizenkeimöl, Pfirsichkernöl, Sesamöl, Avocadoöl, Babassuöl, Hagebuttenöl, Nachtkerzenöl und den flüssigen Anteilen des Kokosöls. Geeignet sind aber auch andere Triglyceridöle, wie synthetische Triglyceridöle, flüssige Paraffinöle aus synthetischen Kohlenwasserstoffen, sowie aus flüchtigen und nichtflüchtigen Siliconölen.

Weiterhin können eingesetzt werden die Di- und Trifettsäureester, insbesondere Trifettsäureester, von gesättigten und/oder ungesättigten linearen und/oder verzweigten Fettsäuren, insbesondere C₆-C₂₂ Fettsäuren mit Glycerin, wie beispielsweise Triglyceride der Caprinsäure und/oder Caprylsäure wie (Neutralöl) bzw. Triglyceride aus konjugierter Linolsäure (CLA) wie Clarinol G-80 von LipidNutrition.

Als natürliche oder synthetische Wachse können erfindungsgemäß eingesetzt werden flüssige Pflanzenwachse wie Jojobawachs.

Erfindungsgemäß ist jede beliebige Kombination der oben genannten Fettstoffe einsetzbar.

Diese Öle können auch allein als kosmetisch/pharmazeutischer Rohstoff dienen.

In einer erfindungsgemäß ganz besonders bevorzugten Ausführungsform enthalten die Nanokapseln ein natürliches pflanzliches Öl, das nach seiner physiologischen Funktion ausgewählt wird: z.B: Aprikosenöl bzw. Olivenöl für die trockene Haut oder Nachtkerzenöl für die empfindliche Haut.

Die Öle und flüssigen Fettstoffe dienen auch als Lösungsmittel für hydrophobe Wirkstoffe wie Retinol in Sonnenblumenöl.

Die erfindungsgemäße Zubereitung kann einen und/oder mehrere lipophile Wirkstoffe enthalten. Als solche Substanzen sind alle Stoffe anzusehen, die sich in einem Öl bei Raumtemperatur oder unter Wärme lösen lassen und kosmetisch und/oder pharmazeutisch relevante Effekte in oder auf der Haut ausüben. Bevorzugt sind diese Aktiva natürlichen Ursprungs, besonders bevorzugt pflanzlichen Ursprungs. Ein Auszug der kosmetisch und/oder dermatologisch wirksamen lipophilen Substanzen, die eingesetzt werden können, kann bestehen aus: Algen- und Pflanzenextrakten (z.B. Phlorogine von Biotechmarine, ein Algenextrakt zur Behandlung von fettiger Haut oder z.B: Incromega V3 von Croda, ein Pflanzenextrakt von Echiomega); Anti-Cellulite wirksame Substanzen (z.B: CLA); Anti-Elastase und Anti-Collagenase wirksame Stoffe (z.B: ungesättigte Fettsäuren wie Ölsäure oder EPA); anti-inflammatorisch wirksame Stoffe (z.B: EPA = Eicosapentaensäure); Antioxidantien (z.B: Salbei-Extrakt von Flavex, Liponsäure und deren Derivate); Ceramide (z.B: verschiedene Ceramide der Firma Cosmoferm); hautberuhigende und hautglättende Wirkstoffe (z.B: Bisabolol); Moisturiser (z.B: Glycerol Monoisostearate, Sucrose Polysoyate); Flavonoide (zu den Flavonoiden gehören Flavanole, Flavanone, Anthocyanidine, Flavone und Flavonole, wie z.B. Sinensetin, oder Polyphenole, wie die im Grüntee oder Trauben); Phytosterole (wie *β*-Sitosterol aus Maisfaseröl); Radikalfänger (z.B: Ubichinol-Derivate wie Coenzym Q10); Saponine (z.B: vom Ginseng, Süßholzwurzel und Rosskastanie); sauerstoffbindende Substanzen (z. B: Perfluordekalin); sebumreduzierende Substanzen (z.B: 10-Hydroxydecansäure); Stoffe, die die Durchblutung und damit die Versorgung der Haut fördern (z.B: Nikotinsäureester); Terpene (kosmetisch und dermatologisch relevante Terpene sind aufgeführt in der Pharmazeutischen Zeitung Heft 22; 2006 von Sebastian Jäger u.a.); Vitamine (Retinol und Derivate, Vitamin E und Derivate wie Tocotrienole oder Carotine und Carotinoide, wie Lycopene, Lutein oder Fucoxanthin, Vitamin D und Derivate);

Alle diese Wirkstoffe sollen die Hautalterung und/oder das Photoageing (durch UV- bzw. Umweltbelastung) verhindern bzw. inhibieren; die Synthese von dermalen und epidermalen Makromolekülen stimulieren bzw. deren Degradation verhindern; die Proliferation von Fibroblasten und Keratinozyten anregen und somit den Schutz und die Erhaltung einer gesunden Haut bewirken.

Die erfindungsgemäße kosmetische oder pharmazeutische Zubereitung für den Hautschutz kann auch weitere Bestandteile als die zuvor genannten enthalten. In einer bevorzugten Ausführungsform enthält sie mindestens eine der aufgezählten Substanzen. Sie kann auch jede beliebige Kombination der bei den Wirkstoffen und Ölen aufgezählten Bestandteile enthalten.

Die erfindungsgemäßen Zubereitungen können weiterhin wenigstens einen Stabilisator, der vorzugsweise ein wasserlöslicher Alkohol ist, enthalten. Der Stabilisator/Solvent wird vorzugsweise in einer Konzentration von 10 - 20 Gew.-%, bezogen auf die gesamte Zubereitung, eingesetzt. Geeignet sind je nach Darreichungsform einwertige Alkohole, wie z. B. Ethanol, Propanol oder Isopropanol. Weiterhin geeignet sind wasserlösliche Polyole. Hierzu zählen wasserlösliche Diole, Triole und höherwertige Alkohole. Unter den Diolen eignen sich C₂ bis C₁₀- Diole insbesondere 1,2-Propylenglycol, 1,2-Butylenglycol, 1,3-Butylenglycol und 1,4-Butylenglycol, 1,2-Pentadiol, 1,6-Hexandiol und 1,2 Octandiol. Weiterhin bevorzugt geeignet sind Glycerin und insbesondere Diglycerin und Triglycerin, 1,2,6-Hexantriol sowie die Dipropylenglycole. Für die Herstellung der erfindungsgemäßen Nanokapseln werden bevorzugt Ethanol oder 1,2 Pentandiol (Hydrolite 5 von Symrise) eingesetzt, in Konzentrationen von 10 bis 30 Gew.-% besonders bevorzugt von 15 - 20 Gew.-% bezogen auf die gesamte Formulierung, sodass somit auf eine andere Konservierung (klassische Konservierungsmittel) verzichtet werden kann. Die erfindungsgemäße Zubereitung kann deshalb als konservierungsmittelfrei deklariert werden.

Die hier beschriebene Erfindung beinhaltet ebenfalls das kolloidale Trägersystem in kosmetisch und dermatologisch interessanten wässerigen Formulierungen (Sprühformulierung, unterschiedlichste Gele, Lotionen und Cremes) sowie deren Verwendung zur Behandlung, zum Schutz und zur Pflege der Haut, Haare, Nägel und Lippen.

Im Folgenden soll das Verfahren zur Herstellung einer erfindungsgemäßen Zubereitung anhand von einigen Ausführungsbeispielen und Abbildungen, die nicht als einschränkend zu verstehen sind, demonstriert werden.

### Beispiel 1

Kolloidales Trägersystem mit Vitamin E und Fischöl gegen Psorriasis
a) 1,4 g Imwitor 375 und 0,6 g Diglyceryl-Monooleate werden unter Wärme in 6 g Ethanol gelöst.
b) 0.05 g Lipochroman-6 (Antioxidants von Lipotec) werden in 2 g Ethanol gelöst. Danach erfolgt die Zugabe von 5 g Fischöl (EPA der Firma Croda, reich an Omega-3 Säuren) und 5 g Tocopherol (Vitamin E der Firma BASF).
   Emulgatorlösung der Ölphase zufügen.
c) Wasserphase vorlegen (29,95 g) und Ölphase unter Homogenisieren zufügen,
   dann Hochdruckhomogenisation für 5 Minuten bei 800 bar.

Es ergeben sich negativ geladene Vesikel (-48 mV) mit einer Teilchengröße von 108 nm.

Die Stabilität der Ölphase wurde überprüft, indem das unverkapselte Öl und das verkapselte Öl für 3 Tage bei 40 Grad gelagert wurden und die Aktivität der Antioxidantien gemessen wurde.

Das reine Öl verliert über den Zeitraum 29% seiner Aktivität, dagegen zeigte sich keine Reduktion in der Aktivität, wenn das Öl im kolloidalen Trägersystem verkapselt vorlag.

Das Trägersystem ist extrem stabil, so zeigt sich keine Solubilisierung der Tröpfchen bei Zugabe von 50% Ethanol.

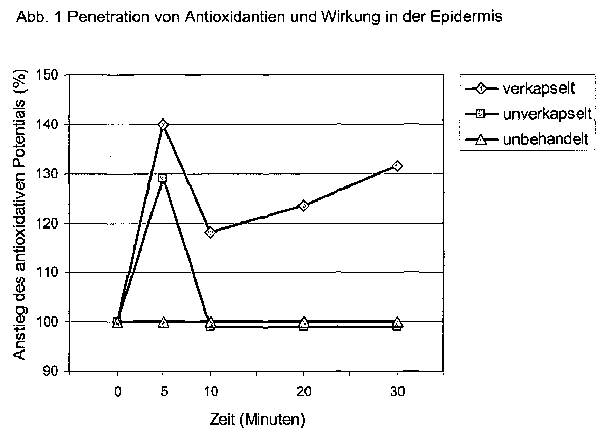

Eine Messung der Penetration der im Öl gelösten Antioxidantien (Vitamin E und Lipochroman) unverkapselt versus Verkapselung im kolloidalen Trägersystem in die Haut wurde mit Hilfe der ESR-Spektroskopie bestimmt. Die Haut hat einen natürlichen anti-oxidativen Schutz, gegeben durch hauteigene enzymatische und nicht-enzymatische Wirksubstanzen. Dieses anti-oxidative Potential der Haut (Skin Antioxidative Potential, SAP) kann gemessen werden, indem die Epidermis mit Testradikalen inkubiert wird und die Reduktion der Radikale nach Penetration der Antioxidatien in die Haut mittels ESR verfolgt wird. Topisch applizierte Antioxidantien, die in die Haut penetrieren können, können dann das SAP erhöhen. Während die unverkapselten Antioxidantien sehr schnell in die Haut eindringen und das SAP erhöhen, werden sie so schnell auch in der Haut verbraucht, und nach 10 Minuten ist wieder der Ausgangszustand hergestellt. Die Nanoemulsion zeigt auch schon nach 5 Minuten eine Erhöhung des SAPs, die signifikant höher ist, dann einen geringen Abfall und wieder einen Anstieg. Über den gesamt getesteten Zeitraum ergibt sich eine signifikante Erhöhung der antioxidativen Kapazität in der Epidermis, die nur mit einer Penetration der Antioxidantien mit Hilfe des kolloidalen Trägersystems zu erklären ist. (Abb.1)

Nanoemulsion mit Retinol gegen Hautalterung
a) 0,1 g Lipochroman-6 (Antioxidans der Firma Lipotec) in 2 g Ethanol lösen und dann 33,3 g Retinol 15S (15% Retinol in Sonnenblumenöl gelöst von BASF) hinzufügen
b) 3,5 g Imwitor 375 und 1,5 g Diglyceryl-Monooleate in 14 g Ethanol lösen, Phase a und b zusammenfügen
c) 45,6 g Wasser vorlegen und Ölphase unter Homogenisation zufügen, dann Hochdruckhomogenisation für 5 Minuten bei 800 bar.

Die entstandenen Retinol-Nanokapseln mit einem Wirkstoffgehalt von 5% reinem Retinol weisen eine Teilchengröße von 170 nm auf und besitzen eine negative Oberflächenladung.

## Patentansprüche

1. Kolloidales Trägersystem in Form einer Nanokapsel, umfassend mindestens die folgenden Bestandteile:
- einen flüssigen Lipidkern,
- eine den Kern umgebende kontinuierliche Schale, enthaltend mindestens einen membranbildenden, natürlichen anionischen Emulgator und mindestens einen einkettigen, natürlichen nicht ionischen Coemulgator, ausgewählt aus der Gruppe von Diglyceryl-Monooleate, Diglyceryl-Monolinoleate, Polyglyceryl-Monooleate, Polyglyceryl-Monolinoleate,
wobei es sich bei dem membranbildenden, natürlichen anionischen Emulgator um Glyceryl Citrate / Lactate / Linoleate / Oleate handelt.

2. Kolloidales Trägersystem nach Anspruch 1,
**gekennzeichnet durch**
eine Teilchengröße von 100 bis 200 nm.

3. Kolloidales Trägersystem nach einem der Ansprüche 1 bis 2,
**dadurch gekennzeichnet,**
**dass** es sich bei dem einkettigen, natürlichen nicht ionischen Coemulgator bevorzugt um Diglyceryl-Monooleate handelt.

4. Kolloidales Trägersystem nach einem der Ansprüche 1 bis 3,
**dadurch gekennzeichnet,**
**dass** der flüssige Lipidkern aus
- einer oder mehreren Ölkomponenten und/oder
- lipophilen Wirkstoffen, die in einem oder mehreren Ölen gelöst sind,
gebildet wird.

5. Zubereitung, umfassend Nanokapseln nach einem der Ansprüche 1 bis 4,
**dadurch gekennzeichnet,**
**dass** sie den flüssigen Lipidkern in einer Menge von 15-50 Gew.-%, bezogen auf die Gesamtmenge der Zubereitung, enthält.

6. Zubereitung nach Anspruch 5, wobei die Zubereitung wässrig ist.

7. Zubereitung nach einem der Ansprüche 5 oder 6,
**dadurch gekennzeichnet,**
**dass** sie weiterhin mindestens einen Stabilisator in Form eines wasserlöslichen Alkohols oder eines Polyols enthält.

8. Zubereitung nach einem der Ansprüche 5 bis 7 zur therapeutischen Behandlung des menschlichen oder tierischen Körpers.

9. Verwendung der Zubereitung nach einem der Ansprüche 5 bis 8 für einen kosmetischen und/oder nicht-therapeutischen, dermatologischen Zweck.

## Claims

1. A colloidal carrier system in the form of a nanocapsule, comprising at least the following components:
- a liquid lipid core,
- a continuous shell surrounding the core, comprising at least one membrane-forming, natural anionic emulsifier and at least one single-chain, natural non-ionic co-emulsifier, selected from the group of diglyceryl-monooleates, diglyceryl-monolinoleates, polyglyceryl-monooleates, polyglyceryl-monolinoleates,
wherein the membrane-forming, natural anionic emulsifier is glyceryl citrate/lactate/linoleate/oleate.

2. The colloidal carrier system according to claim 1,
**characterized by**
a particle size of 100 to 200 nm.

3. The colloidal carrier system according to any one of claims 1 to 2,
**characterized in that**
the single-chain, natural non-ionic co-emulsifier is preferably diglyceryl-monooleates.

4. The colloidal carrier system according to any one of claims 1 to 3,
**characterized in that**
the liquid lipid core is formed from
- one or more oil components and/or
- lipophilic agents which are dissolved in one or more oils.

5. A preparation, comprising nanocapsules according to any one of claims 1 to 4,
**characterized in that**
it comprises the liquid lipid core in an amount of 15-50 % by weight based on the total amount of the preparation.

6. The preparation according to claim 5, wherein the preparation is aqueous.

7. The preparation according to any one of claims 5 or 6,
**characterized in that**
it further comprises at least one stabilizer in the form of a watersoluble alcohol or a polyol.

8. The preparation according to any one of claims 5 to 7 for therapeutically treating the human or animal body.

9. Use of the preparation according to any one of claims 5 to 8 for a cosmetic and/or non-therapeutic, dermatologic purpose.

## Revendications

1. Système support colloïdal sous la forme d'une nanocapsule, comprenant au moins les composants suivants :
- un noyau lipidique liquide,
- une enveloppe continue entourant le noyau, comprenant au moins un émulsifiant anionique naturel formant membrane et au moins un co-émulsifiant naturel non ionique à chaîne unique sélectionné dans le groupe de monooléates de diglycéryle, de monolinoléates de diglycéryle, de monooléates de polyglycéryle, de monolinoléates de polyglycéryle,
dans lequel l'émulsifiant anionique naturel formant membrane est de citrate/lactate/linoléate/oléate de glycéryle.

2. Système support colloïdal selon la revendication 1,
**caractérisé par**
une taille de particules de 100 à 200 nm.

3. Système support colloïdal selon l'une quelconque des revendications 1 à 2,
**caractérisé en ce que**
de préférence, le co-émulsifiant non ionique naturel à chaîne unique est de monooléates de diglycéryle.

4. Système support colloïdal selon l'une quelconque des revendications 1 à 3,
**caractérisé en ce que**
le noyau lipidique liquide est formé
- d'un ou plusieurs composants d'huile et/ou
- d'agents actifs lipophiles qui sont dissous dans une ou plusieurs huiles.

5. Préparation comprenant des nanocapsules selon l'une quelconque des revendications 1 à 4,
**caractérisée en ce que**
la préparation comprend le noyau lipidique liquide en une quantité de 15 à 50 % en poids par rapport à la quantité totale de la préparation.

6. Préparation selon la revendication 5, la préparation étant aqueuse.

7. Préparation selon l'une quelconque des revendications 5 ou 6,
**caractérisée en ce que**
la préparation comprend en outre au moins un stabilisateur sous la forme d'un alcool soluble dans l'eau ou d'un polyol.

8. Préparation selon l'une quelconque des revendications 5 à 7 pour le traitement thérapeutique du corps humain ou animal.

9. Utilisation de la préparation selon l'une quelconque des revendications 5 à 8 dans un but dermatologique cosmétique et/ou non thérapeutique.
